# EUROPEAN PATENT APPLICATION

(11) **EP 4 445 887 A1**
(43) Date of publication of application: **16.10.2024**
(21) Application number: 23171775.2
(22) Date of filing: 05.05.2023
(51) Int. Cl.: A61F 13/475, A61F 13/49, A61F 13/532, A61F 13/533, A61F 13/536

(54) **ABSORBENT ARTICLES WITH CHANNELED ABSORBENT CORES**

(30) Priority: 11.04.2023 US 202363495553 P
(71) Applicant: Laboratorios Indas, S.A.U., 28223 Madrid (ES)
(72) Inventor: Bernabé Corral, Belén, 28223 Madrid (ES); García Cruz, Tomás, 28223 Madrid (ES)
(74) Representative: Lohr, Jöstingmeier & Partner

(57) **Abstract**

The present disclosure describes absorbent articles including absorbent cores. An absorbent core is positioned between a liquid-permeable topsheet and a liquid-impermeable backsheet and includes a central channel and one or more lateral channels extending from the central channel. The channels are substantially free of absorbent material. The absorbent core may include embossed elements and/or regions and the channels may be positioned between the embossed regions. Some implementations include an additional absorbent core positioned between the absorbent core and the topsheet. The additional absorbent core includes one or more embossed pathways. The embossed pathways of the additional absorbent core may overlie the channels of the absorbent core.

## Description

### Field of the invention

The present invention relates generally to absorbent products like incontinence briefs, protective underwear, pads, diapers, training pants, and the like; and more particularly, but not by way of limitation, to absorbent articles having absorbent cores with channels substantially free of absorbent material.

### Description of the related art

Absorbent products can include, for example, disposable absorbent articles that are wearable by a user, examples of which include diapers, training pants, incontinence briefs, protective underwear, and pads, such as feminine hygiene pads and shaped absorbent pads, all of which may be made in disposable forms. "Disposable" refers to articles that are designed to be discarded after a limited use rather than being laundered or otherwise restored for reuse. Disposable absorbent products have met with widespread acceptance in the marketplace for a variety of applications, including infant and adult incontinence care, in view of the manner in which such products can provide effective and convenient liquid absorption and retention while maintaining the comfort of the wearer. Such disposable absorbent articles often include a topsheet that is configured to be closest to the wearer during use, a liquid impermeable backsheet or outer cover, and an absorbent core located between the topsheet and the backsheet. In some instances, such disposable absorbent articles also include an acquisition-distribution layer (ADL) disposed between the topsheet and the absorbent core, in addition to other layers for distribution, containment, reinforcement, etc., that may be located above or below the absorbent core. Elasticated standing leg cuffs and leg gathers are also often used in such articles to provide improved fit and reduced leakage around a wearer's legs, relative to articles without such cuffs or gathers.

U.S. Patent No. 4,670,011 discloses certain prior art examples of diapers, and U.S. Patents No. 6,976,978 and No. 4,940,464 disclose certain prior art examples of disposable incontinence garments or training pants.

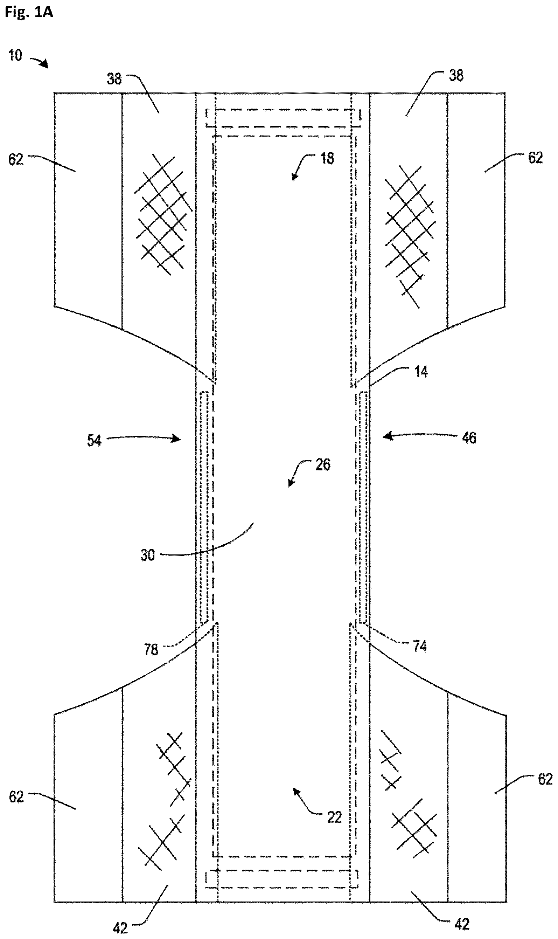

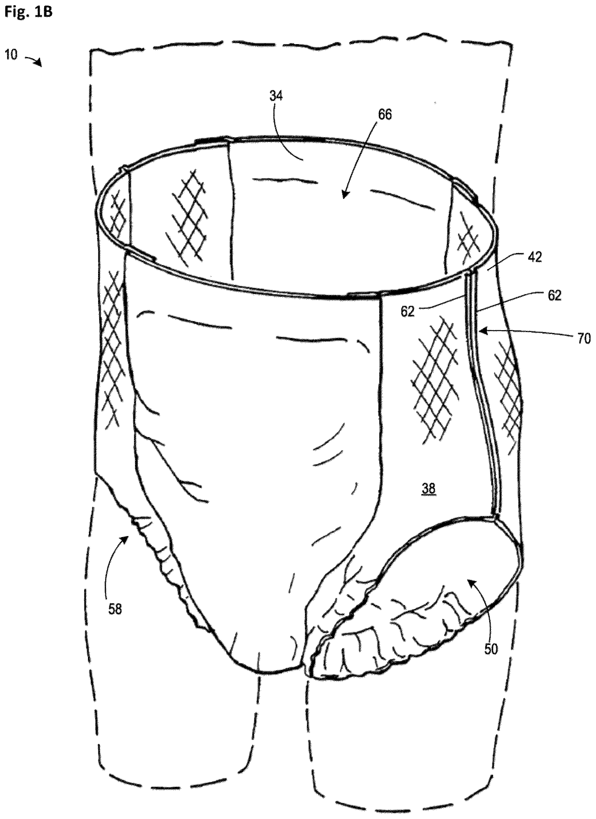

One example of such a disposable absorbent article is shown in FIGS. 1A and 1B, which depict a lower plan view and a perspective view, respectively, of adult protective underwear 10. Underwear 10 includes a chassis 14 having a front waist portion 18, an opposing rear waist portion 22, and a crotch portion 26 extending longitudinally between front and rear waist portions 18, 22. Chassis 14 further includes a backsheet 30 defining an outer surface and configured to face away from a wearer during use of the underwear, and topsheet 34 defining an opposing body facing surface and configured to face a wearer during use of the underwear.

As shown in FIGS. 1A and 1B, underwear 10 further includes a pair of front elastic side panels 38 and a pair of rear elastic side panels 42 configured to couple rear waist portion 22 to front waist portion 18 in a well-known configuration in which a left side 46 of the chassis defines a first leg opening 50 for a wearer's left leg, and in which a right side 54 of the chassis defines a second leg opening 58 for the wearer's right leg. In the depicted configuration, each of side panels 38, 42 includes a connection portion 62 configured to be coupled to a connection portion 62 of another of side panels 38, 42. Specifically, connection portion 62 of the left one of front side panels 38 is configured to be coupled to connection portion 62 of the left one of rear side panels 42, and connection portion 62 of the right one of front side panels 38 is configured to be coupled to connection portion 62 of the right one of rear side panels 42, such that the waist portions 18, 22 and side panels, 38, 42 cooperate to define a waist opening 66 as shown in FIG. 1B. Connection portions 62 of the respective side panels can be permanently coupled together to define a tear-able side seam 70, such as, for example, via adhesive, ultrasonic, or thermal bonds. Such tear-able side seams generally cannot be refastened, and thereby render an article unusable once opened. Alternatively, connection portions 62 of the respective side panels can be removably coupled to define a refastenable or adjustable side seam, such as, for example, via hook-and-loop fasteners. Hook and loop fasteners are mechanical fasteners that include hooks, such as in a hook fastener portion, that are configured to engage loops in a loop fastener portion or in fibers of a sheet of fabric; for example, a nonwoven or woven fabric with fibers that define open or loop-like regions into which the hooks can extend and engage. Examples of such hook and loop fasteners may be referred to as VELCRO.

As is known in the art, underwear 10 can include one or more elastic elements coupled to the chassis such that the one or more elastic elements resist expansion of a circumference of the first leg opening and resist expansion of a circumference of the second leg opening. For example, as shown in FIG. 1A, the depicted embodiment of the chassis (14) includes a first elastic region 74 along left side 46, and a second elastic region 78 along right side 54. In some configurations, elastic regions 74, 78 can each be defined by one or more elastic strands, which may be referred to in the art as "leg elastics," coupled to the chassis, for example laminated between the topsheet or an additional leg cuff layer and the backsheet. In other configurations, elastic regions 74, 78 can each be defined by an elastic film coupled to the chassis, for example laminated between the topsheet and the backsheet. In configurations in which elastic regions 74, 78 are defined by elastic film, the regions can be defined by separate pieces of elastic film or by separate regions of a single piece of elastic film. As shown in FIG. 1A, elastic regions 74, 78 may be parallel to and/or extend along a majority of a length of each of sides 46 and 54, provided that the elastic regions are configured to provide a biasing force that resists expansion of the leg openings when the chassis is in its closed configuration and tends to contract the leg opening around a wearer's leg, as shown in FIG. 1B. Contraction of the leg opening to conform to the wearer's leg is desired for good containment of urine and feces in an absorbent product.

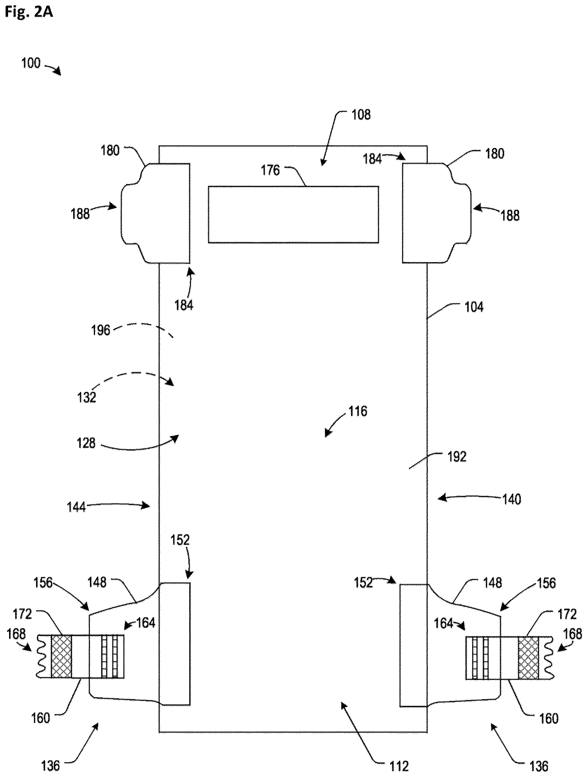

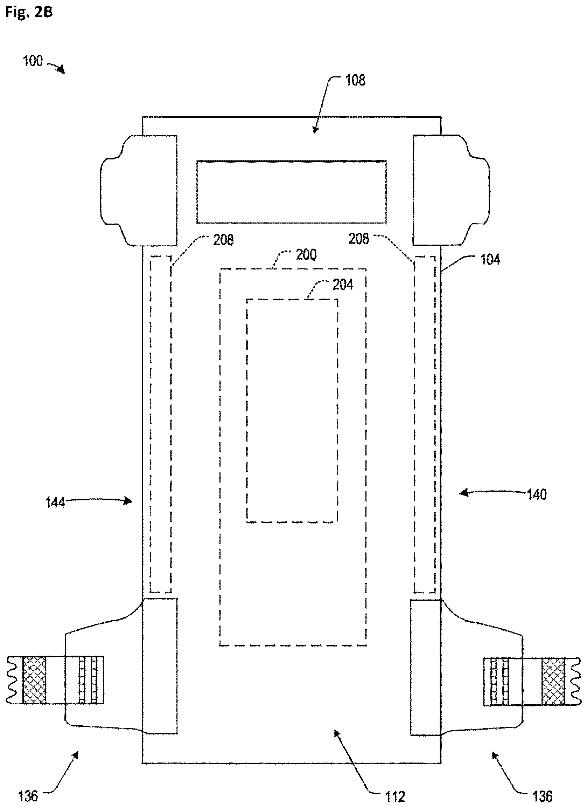

Another example of such a disposable absorbent article is shown in FIGS. 2A and 2B, which depict lower plan views of a diaper 100. Diaper 100 includes a chassis 104 having a front waist portion 108, an opposing rear waist portion 112, and a crotch portion 116 extending longitudinally between front and rear waist portions 108, 112. Chassis 104 further includes an outer surface 128 configured to face away from a wearer during use of the diaper, and an opposing body facing surface 132 configured to face a wearer during use of the diaper. In the view of FIG. 2A, a dashed leader extends from the body facing surface to reference numeral 132 because body facing surface 132 is opposite outer surface 128 and therefore not visible in the view of FIG. 2A.

As shown in FIG. 2A, diaper 100 further includes a pair of closure members 136 configured to couple rear waist portion 112 to front waist portion 108 in a well-known configuration in which a left side 140 of the chassis defines a first leg opening for a wearer's left leg, and in which a right side 144 of the chassis defines a second leg opening for the wearer's right leg, similar in some respects to what is shown in FIG. 1B for underwear 10 (or a training pant). In the depicted configuration, the closure members include a pair of back ears or back ear panels 148 each having a first end 152 bonded to rear waist portion 112 of chassis 104, and a second end 156 shown extending away from rear waist portion 112. "Bonded" refers to the joining, adhering, connecting, attaching, or the like, of two elements via adhesive(s), ultrasonic bond(s), and/or thermal bond(s). Two elements will be considered to be bonded together when they are bonded directly to one another or indirectly to one another, such as when each is directly bonded to intermediate elements.

Each closure member 136 further includes a fastener tab 160 with a first end 164 bonded to back ear 148, a second end 168 shown extending laterally outward from back ear 148, and a fastener portion 172 coupled to the fastener tab. Back ears 148 are each formed of a stretchable elastic material, such as a nonwoven laminate, that permit adjustment in the width and tension of back ears 148 to vary the form and fit of diaper 100 when worn by a user.

Fastener tabs 160 are formed of an inelastic nonwoven material and carry fastener portions 172. Fastener portions 172 can include strips of hook material configured to interact with a corresponding loop material in the well-known hook-and-loop fastener arrangement. Connection of closure members 136 to front waist portion 108 is facilitated by a landing zone 176 configured to be engaged by fastener portions 172. In this embodiment, landing zone 176 is defined by an anchoring member that includes a strip of loop material bonded to front waist portion 108 of chassis 104, for example, to the backsheet, and configured to be engaged by the hook material of fastener portions 172.

As shown in FIG. 2A, diaper 100 also includes a pair of front ears 180 extending from opposite sides 140, 144 of chassis 104 with each of front ears 180 having a first end 184 bonded to front waist portion 108 of chassis 104, and a second end 188 shown extending away from a respective side of front waist portion 108. Front ears 180 are each formed of a relatively soft nonwoven material and are each configured for grasping by the caregiver during application of the diaper, as well as to be overlapped by the corresponding fastener tab 160 and/or back ear 148 to prevent the edges of fastener tab 160 from pinching, rubbing, or otherwise irritating a user's skin in use when fastening portions 172 are engaged with landing zone 176 to couple rear waist portion 112 to front waist portion 108. In some embodiments, front ears 180 include loop fastener portions or a fabric that is configured to be engaged by hook fastener portions such that fastener portions 172 can engage front ears 180.

Outer surface 128 is defined by a liquid-impermeable backsheet 192 that defines outer surface 128, and a liquid-permeable topsheet 196 that defines body facing surface 132 and is configured to be closest to the wearer during use. "Liquid impermeable," when used in describing a layer or multi-layer laminate, means that a liquid, such as urine, will not pass through the layer or laminate, under ordinary use conditions, in a direction generally perpendicular to the plane of the layer or laminate at the point of liquid contact. "Lamination" is the technique of manufacturing a material in multiple layers, so that the composite material has benefits of all the combined layers, such as, for example, improved mechanical strength or durability, improved stability, lower permeability to water, and/or other properties. A laminate includes two or more layers of material(s) that are permanently assembled by heat, pressure, ultrasonic welding, or adhesives.

As shown in FIG. 2B, the depicted embodiment includes an absorbent core 200 disposed between topsheet 196 and backsheet 192. An "absorbent core" is a structure typically disposed between a topsheet and backsheet of an absorbent article and containing materials like super absorbent particles (SAP) and/or cellulosic fibers that are configured to absorb liquid in the absorbent article. Absorbent core 200 may also be contained by a core cover that extends over the top and/or bottom sides of absorbent core 200 and may be continuous or sealed along the edges of absorbent core 200.

As shown in FIG. 2B, diaper 100 also includes an acquisition-distribution layer (ADL) 204 disposed between the topsheet and the absorbent core. "Layer" when used in the singular can be a single element or a plurality of elements. For example, a plurality of sheets may together define a single layer, such as, for example, a layer with a particular function to which the sheets of the layer contribute. Additional layers for fluid management, reinforcement, and performance enhancement may be incorporated inside the core cover or outside the core cover and between topsheet 196 and backsheet 192.

As is known in the art, diaper 100 can include one or more elastic elements coupled to the chassis such that the one or more elastic elements resist expansion of a circumference of the first leg opening and resist expansion of a circumference of the second leg opening. For example, as shown in FIG. 2B, the depicted configuration of the chassis (104) includes a first elastic region 208 along left side 140, and a second elastic region 208 along right side 144. In some configurations, elastic regions 208 can each be defined by one or more elastic strands, which may be referred to in the art as "leg elastics," coupled to the chassis, for example laminated between the topsheet (or an additional leg cuff layer) and the backsheet. In other configurations, elastic regions 208 can each be defined by an elastic film coupled to the chassis, for example laminated between the topsheet (or an additional leg cuff layer) and the backsheet. In configurations in which elastic regions 208 are defined by elastic film, the regions can be defined by separate pieces of elastic film or by separate regions of a single piece of elastic film. As shown in FIG. 2B, elastic regions 208 may be parallel to and/or extend along a majority of a length of each of sides 140 and 144, provided that the elastic regions are configured to provide a biasing force that resists expansion of the leg openings when the chassis is in its closed configuration.

Diaper 100 of FIGS. 2A and 2B is typically packaged and sold in a folded and unfastened configuration in which chassis 104 is folded in half such that rear waist portion 112 overlaps front waist portion 108, but fastener portions 172 do not engage landing zone 176. While diaper 100 is described and/or illustrated as a baby diaper, diaper 100 can also be or comprise an incontinence brief, protective underwear, pad, training pant, and the like.

Some conventional absorbent articles use randomly dispersed SAP and fiber loosely bonded to the backsheet and the topsheet materials using adhesives or a similar type of air form construction placed within a core cover or an envelope. In conventional absorbent articles, the absorbent core may not easily take a contoured shape of the user, causing fit issues. Additionally, materials shifted and bunched during use, which allowed absorbent materials to migrate within the absorbent core to areas outside or away from insult zones. Allowing the absorbent material to migrate away from the insult zones may compromise the effectiveness of the absorbent core at absorbing fluid insults. Moreover, in conventional absorbent articles, the absorbent core may not easily direct fluids to the front and sides of the absorbent core, which may result in concentration of fluids on or near the rear portion of the absorbent core and/or article and non-used portions of the absorbent core and/or absorbent article on or near the front portion of the absorbent core and/or article.

### Summary of the invention

An object of the invention is to mitigate the above explained deficiencies, while keeping cost for the absorbent articles competitive. The invention is defined by the independent claim 1. Advantageous embodiments are subjects of the dependent claims.

The present disclosure relates to absorbent articles, such as incontinence briefs, protective underwear, pads, diapers, training pants, and the like, that direct some fluids to generally non-used portions of an absorbent core and/or the absorbent article and that reduce the likelihood or prevent the migration of absorbent materials in an absorbent core. The absorbent article can include an absorbent core with a central channel that extends longitudinally along the absorbent core and one or more lateral channels that extend laterally outward from a point on the central channel toward a side edge of the absorbent core and longitudinally toward an end of the absorbent core. The channels are at least substantially free of absorbent material. Because the channels are at least substantially free of absorbent material, the channels provide pathways for channeling fluids transversely and longitudinally through the absorbent core. For example, the channels may direct some fluids to the front and sides of the absorbent core and/or absorbent article, which may improve fluid distribution and leakage protection of the absorbent article. The absorbent article can also include an absorbent core with embossed regions and/or elements. The embossed regions and/or elements may help manage and disperse fluid flow and direct fluid to the channels.

The absorbent article may comprise a chassis and/or an absorbent first core. The first core may have a longitudinal extension. For example, the chassis may have at least one of a front waist portion, a rear waist portion, and a crotch portion between the front and rear waist portions. In some such configurations, the chassis has a length extending from a front waist edge of the front waist portion to a rear waist edge of the rear waist portion. Preferably, the chassis may comprise a liquid-permeable topsheet and a liquid-impermeable backsheet. The first core may be disposed between the topsheet and the backsheet. The first core may comprise a mixture of cellulosic fibers, super-absorbent polymer (SAP), and adhesive. In some configurations, the first core has at least one of a front end, a rear end, two side edges, a length extending longitudinally between the front end and the rear end, a front half between a longitudinal midpoint of the first core and the front end, and a rear half between the longitudinal midpoint of the first core and the rear end. The first core may define a central channel and a number of lateral channels, for example two lateral channels. Preferably, the optional central channel extends longitudinally from the rear half to the front half. The two lateral channels may be in the front half of the first core. In some configurations, the lateral channels extend from a common point on the central channel toward respective ones of the side edges of the first core. In some configurations, the first core is not enclosed in a core cover within the topsheet and backsheet.

Preferably, the optional (e.g., two) lateral channels are front lateral channels extending longitudinally toward the front end of the first core.

The common point is preferably within a confluence region. Only to provide an example, the confluence region may have a first transverse dimension of from 20 to 60 millimeters. Preferably, the confluence region is circular. In some such configurations, the first transverse dimension is hence a diameter. The confluence region may be located in the front half of the first core. In some such configurations, the confluence region has a center that is 10 to 30 millimeters from the longitudinal midpoint of the first core.

The absorbent article may further comprise an absorbent second core. For example, the second core is disposed between the first core and the topsheet. Preferably, the second core covers at least a portion of the central channel and/or a portion of the two lateral channels. The second core may comprise an embossed first pattern.

The optional embossed first pattern may have one or more embossed linear paths extending longitudinally from a rear half of the second core to a front half of the second core. Preferably, at least a portion of at least one of the embossed linear paths overlies at least a portion of the central channel.

The optional embossed first pattern may have an embossed central path and/or a number (e.g., two) embossed lateral paths. For example, the embossed central path may extend longitudinally from a rear half of the second core to a front half of the second core. The number (e.g., two) embossed lateral paths may extend from a common point on the optional embossed central path toward respective ones of side edges of the second core. Preferably, at least a portion of the embossed central path overlies at least a portion of the central channel and/or at least a portion of each of the embossed lateral paths overlies at least a portion of a corresponding one of the lateral channels. For example, the two embossed lateral paths may be embossed front lateral paths extending from the common point toward a front end of the second core. Preferably, the embossed first pattern may further comprise two embossed rear lateral paths extending from a rear common point on the embossed central path toward a rear end of the second core.

The absorbent article may further comprise a storage core. The optional storage core may for example be disposed between the first core and the backsheet.

The first core may further comprise one or more embossed linear paths extending longitudinally from a rear half of the first core to a front half of the first core.

Preferably, the topsheet comprises a marking having a second pattern. In some such configurations, at least a portion of the second pattern is substantially aligned with at least a portion of the central channel and at least a portion of the two lateral channels.

For example, at least one of the central channel and the two lateral channels may have a transverse dimension of from 10 to 25 millimeters. In some such configurations, the transverse dimension is of 25 millimeters (mm), at least within an error margin of ±2 mm, preferably ±1 mm).

Preferably, at least one of an embossed linear path, an embossed central path, and an embossed lateral path has a transverse dimension of from 3 to 8 millimeters.

The absorbent article may further comprise an acquisition-distribution layer (ADL). In some such configurations, the optional ADL is disposed between the topsheet and the first core. Preferably, the ADL may overlie the common point on the central channel. In some such configurations, a center of the ADL overlies the common point on the central channel. The ADL may have a longitudinal extension. As an example, the longitudinal dimension of the ADL may be of less than 200 millimeters. In some such configurations, the longitudinal dimension of the ADL is of from 120 to 170 millimeters. Preferably, the longitudinal dimension of the ADL is of more than 75 percent smaller than the longitudinal dimension of the first core. In some such configurations, the longitudinal dimension of the ADL is of more than 78 percent smaller than the longitudinal dimension of the first core.

As used herein, various terminology is for the purpose of describing particular implementations only and is not intended to be limiting of implementations. For example, as used herein, an ordinal term (e.g., "first," "second," "third," etc.) used to modify an element, such as a structure, a component, an operation, etc., does not by itself indicate any priority or order of the element with respect to another element, but rather merely distinguishes the element from another element having a same name (but for use of the ordinal term). The term "coupled" is defined as connected, although not necessarily directly, and not necessarily mechanically; two items that are "coupled" may be unitary with each other. The terms "a" and "an" are defined as one or more unless this disclosure explicitly requires otherwise. The term "substantially" is defined as largely but not necessarily wholly what is specified - and includes what is specified; e.g., substantially 90 degrees includes 90 degrees and substantially parallel includes parallel - as understood by a person of ordinary skill in the art. In any disclosed embodiment, the term "substantially" may be substituted with "within [a percentage] of" what is specified, where the percentage includes 0.1, 1, 5, and 10 percent; and the term "approximately" may be substituted with "within 10 percent of" what is specified. The phrase "and/or" means "and" or "or." To illustrate, A, B, and/or C includes: A alone, B alone, C alone, a combination of A and B, a combination of A and C, a combination of B and C, or a combination of A, B, and C. In other words, "and/or" operates as an inclusive or. The phrase "A, B, C, or a combination thereof" includes A alone, B alone, C alone, a combination of A and B, a combination of A and C, a combination of B and C, or a combination of A, B, and C. In other words, "or" is used inclusively unless otherwise is expressly specified.

The terms "comprise" and any form thereof such as "comprises" and "comprising," "have" and any form thereof such as "has" and "having," and "include" and any form thereof such as "includes" and "including" are open-ended linking verbs. As a result, an apparatus that "comprises," "has," or "includes" one or more elements possesses those one or more elements, but is not limited to possessing only those elements. Likewise, a method that "comprises," "has," or "includes" one or more steps possesses those one or more steps, but is not limited to possessing only those one or more steps.

Any implementation of any of the apparatuses, systems, and methods can consist of or consist essentially of - rather than comprise/include/have - any of the described steps, elements, and/or features. Thus, in any of the claims, the term "consisting of" or "consisting essentially of" can be substituted for any of the open-ended linking verbs recited above, in order to change the scope of a given claim from what it would otherwise be using the open-ended linking verb. Additionally, it will be understood that the term "wherein" may be used interchangeably with "where."

Further, a device or system that is configured in a certain way is configured in at least that way, but it can also be configured in other ways than those specifically described. Aspects of one example may be applied to other examples, even though not described or illustrated, unless expressly prohibited by this disclosure or the nature of a particular example. Some details associated with the aspects described above and others are described below.

Some details associated with the aspects are described above, and others are described below. Other implementations, advantages, and features of the present disclosure will become apparent after review of the entire application, including the following sections: Brief Description of the Drawings, Detailed Description, and the Claims.

### Description of Drawings

The following drawings illustrate by way of example and not limitation. For the sake of brevity and clarity, every feature of a given structure is not always labeled in every figure in which that structure appears. Identical reference numbers do not necessarily indicate an identical structure. Rather, the same reference number may be used to indicate a similar feature or a feature with similar functionality, as may non-identical reference numbers.
- FIG. 1A: is a bottom plan view of a prior art disposable absorbent article, specifically adult protective underwear, in an open configuration.
- FIG. 1B: is a perspective view of the protective underwear of FIG. 1A in a closed configuration.
- FIG. 2A: is a bottom plan view of a prior art disposable absorbent article, specifically a diaper, in an open configuration.
- FIG. 2B: is a bottom plan view of the diaper of FIG. 2A, in an open configuration, showing certain internal components of the diaper.
- FIG. 3: is a bottom plan view of a first example of an absorbent article according to some aspects of this disclosure.
- FIG. 4: is a bottom plan view of a second example of an absorbent article according to some aspects of this disclosure.
- FIG. 5A: is a top plan view of an example of an absorbent core according to some aspects of this disclosure.
- FIG. 5B: is a top plan view of a first example of an absorbent core assembly according to some aspects of this disclosure.
- FIG. 6A: is a top plan view of a second example of an absorbent core assembly according to some aspects of this disclosure.
- FIG. 6B: is a top plan view of a third example of an absorbent core assembly according to some aspects of this disclosure.
- FIG. 7: is an exploded view of a third example of an absorbent article according to some aspects of this disclosure.
- FIG. 8A: is a top plan view of a fourth example of an absorbent core assembly according to some aspects of this disclosure.
- FIG. 8B: is a top plan view of a fifth example of an absorbent core assembly according to some aspects of this disclosure.
- FIG. 8C: is a top plan view of a sixth example of an absorbent core assembly according to some aspects of this disclosure.
- FIG. 8D: is a top plan view of a seventh example of an absorbent core assembly according to some aspects of this disclosure.
- FIG. 8E: is a top plan view of an eight example of an absorbent core assembly according to some aspects of this disclosure.

### Detailed Description of illustrative aspects

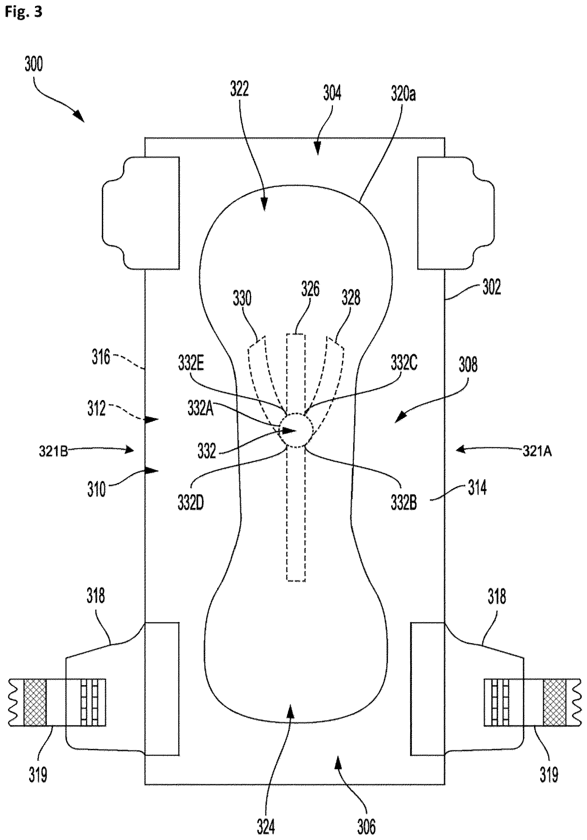

Referring to FIG. 3, an absorbent article 300 is shown. Absorbent article 300 may be a disposable absorbent article, such as an incontinence brief, protective underwear, pad, diaper, training pant, and the like, as illustrative, non-limiting examples.

Absorbent article 300 preferably includes a chassis 302 having at least one of a front waist portion 304, an opposing rear waist portion 306, and a crotch portion 308 extending longitudinally between front waist portion 304 and rear waist portion 306. Chassis 302 may further include an outer surface 310 configured to face away from a wearer during use of absorbent article 300, and/or an opposing body facing (or inner) surface 312 configured to face a wearer during use of absorbent article 300.

Outer surface 310 is preferably defined by a liquid-impermeable backsheet 314. A liquid permeable topsheet 316 may define a body facing surface 312 and is preferably configured to be closest to the wearer during use.

"Liquid impermeable," when used in describing a layer or multi-layer laminate, means that a liquid, such as urine, will not pass through the layer or laminate, under ordinary use conditions, in a direction generally perpendicular to the plane of the layer or laminate at the point of liquid contact. "Lamination" is the technique of manufacturing a material in multiple layers, so that the composite material has benefits of all the combined layers, such as, for example, improved mechanical strength or durability, improved stability, lower permeability to water, and/or other properties. A laminate includes two or more layers of material(s) that are permanently assembled by heat, pressure, ultrasonic welding, or adhesive. Backsheets are typically liquid-impermeable and can include, for example, an inner liquid-impermeable film and an outer nonwoven backsheet that can be a nonwoven fabric. A "film" is a membrane-like layer of material formed of one or more polymers, which does not have a form consisting predominately of a web-like structure of fibers and/or other fibers. Preferably, the backsheet can be breathable, for example, an inner liquid-impermeable film of the backsheet can include a breathable film. The terms "breathable," "breathable film," "breathable laminate" or "breathable outer cover material" or "breathable backsheet" refer to a film, laminate, or outer cover material having a water vapor transmission rate ("WVTR") of at least about 300 grams/meter2/24 hours. Breathable materials typically rely on molecular diffusion of vapor and are substantially liquid impermeable. "Nonwoven backsheet" is a backing substrate layer in the outer cover; a nonwoven backsheet is most often a nonwoven layer facing away from the wearer.

Absorbent article 300 may include back ear panels 318. Optional first ends of back ear panels 318 may be bonded to rear waist portion of 306 of chassis 302 and second ends of back ear panels 318 may preferably extend away from rear waist portion 306. "Bonded" refers to the joining, adhering, connecting, attaching, or the like, of two elements via adhesive(s), ultrasonic bond(s), and/or thermal bond(s). Two elements will be considered to be bonded together when they are bonded directly to one another or indirectly to one another, such as when each is directly bonded to intermediate elements. At least one, preferably each of the optional back ear panels 318 may be formed of a stretchable elastic material, such as a nonwoven laminate, that permits adjustment in the width and tension of back ear panels 318 to vary the form and fit of absorbent article 300 when worn by a user.

At least one, preferably each back ear panel 318 may include a fastener tab 319 with a first end bonded to the corresponding back ear panel 318 and/or a second end that extends laterally outward from the corresponding back ear panel 318. Fastener tabs 319 can each include fastener portions, such as a hook material configured to interact with a corresponding loop material, an adhesive, or another type of fastener. Fastener tabs 319 are configured to be engaged by front waist portion 304, such as by front ear panels or a landing zone. Although back ear panels 318 and fastener tabs 319 are included in the illustrated example, in other implementations, absorbent article 300 may be and/or include a diaper, training pant, protective underwear, incontinence brief, pad, or the like, which may not include back ear panels 318 and fastener tabs 319.

Absorbent article 300 may also include an absorbent core 320a. The absorbent core 320a may be disposed between topsheet 316 and backsheet 314.

Absorbent core 320a may be configured to longitudinally extend along crotch portion 308. A longitudinal length of absorbent core 320a can be, for example, greater than or equal to, or between two of, 150, 180, 210, 240, 270, 300, 330, 360, 390, 420, 450, 480, 510, 540, 560, 590, 620, 650, 680, 710, 740, 770, 800, 830, 860, 890, or 920 millimeters (e.g., between 486 and 755 millimeters), as non-limiting examples. Absorbent core 320a can be coupled to crotch portion 308 and can, but need not, extend longitudinally along the entire length of crotch portion 308. For example, absorbent core 320a can have a longitudinal length at least, or between any two of, 10%, 20%, 30%, 40%, 50%, 60%, 70%, 80%, or 90% smaller than a length of crotch portion 308, and optionally be positioned closer to front waist portion 304 than to rear waist portion 306. Absorbent core 320a may include a front portion 322 and/or a rear portion 324. Front portion 322 may include a front end and/or rear portion 324 may include a rear end. Absorbent core 320a may include a front half that extends from a longitudinal midpoint of absorbent core 320a to a front end of absorbent core 320a and/or a rear half that extends from a longitudinal midpoint of absorbent core 320a to a rear end of absorbent core 320a. Absorbent core 320a may include a left edge near a left side 321A and a right edge near a right side 321B. In the shown example, the left side corresponds to the side for a wearer's left leg when the absorbent article is in a wearable configuration; and the right side corresponds to the side for a wearer's right leg when the absorbent article is in a wearable configuration. Front portion 322 may be, or may include, a front half of absorbent core 320a; and rear portion 324 may be, or may include, a rear half of absorbent core 320a.

As shown, absorbent core 320a may preferably include a central channel 326 that may include at least one pathway extending longitudinally along at least one-fourth (e.g., 25%, 35%, 50%, 60%, 70%, or more) of the length of absorbent core 320a. In the implementation illustrated in FIG. 3, central channel 326 optionally includes a single pathway that extends longitudinally along absorbent core 320a. Central channel 326 may be, as shown in the depicted example, centered relative to the side edges of absorbent core 320a. In this example, central channel 326 is substantially free of absorbent material. As used herein, "substantially free" of absorbent materials means that central channel 326 includes one of, or between any two of, 0%, 1%, 2%, 3%, 4%, 5%, 6%, 7%, 8%, 9%, or 10% absorbent materials, such as SAP or fibers. Central channel 326 may be configured to provide a pathway for channeling fluids longitudinally through absorbent core 320a. At least a portion of central channel 326 may be disposed adjacent to a wearer's groin area. For example, at least a portion of central channel 326 may be adjacent to a wearer's groin area when absorbent article 300 is worn.

Absorbent core 320a may also include one or more lateral channels. Like in the depicted example, absorbent core 320a may include first and second lateral channels 328, 330. As shown, first and second lateral channels 328, 330 may extend along respective curved paths that extend laterally outward from a point on central channel 326 toward a respective side edge of absorbent core 320a, and/or extend longitudinally toward the front end of absorbent core 320a. As shown, the first lateral channel 328 and second lateral channel 330 may extend from a common point on central channel 326. First and second lateral channels 328, 330 can be referred to as front lateral channels because the majority of their respective lengths is in the front half of absorbent core 320a. As described above for central channel 326, first and second lateral channels 328, 330 are substantially free of absorbent material and are configured to provide pathways for channeling fluids transversely (laterally) and longitudinally through absorbent core 320a to distribute those fluids to additional areas of absorbent material within the core. In the depicted example, in use, first and second lateral channels 328, 330 direct fluids toward the right and left sides of front portion 322 of absorbent core 320a. By directing fluids longitudinally and laterally, first and second lateral channels 328, 330 improve fluid distribution along absorbent core 320a and enhance the leakage protection of absorbent article 300 because relatively more liquid will be absorbed by the absorbent material of the core (as opposed to running off of the core if more liquid is applied over a smaller area at a rate that is faster than the absorbent material in that area can absorb).

Central channel 326, first lateral channel 328, and second lateral channel 330 may have the same, similar, or different dimensions. A lateral dimension (e.g., width) of central channel 326, first lateral channel 328, and/or second lateral channel 330 can be, for example, greater than or equal to, or between two of, 10, 15, 20, 25, 30, 35, 40 millimeters (e.g., between 15 and 30 millimeters), as non-limiting examples. The channels may also vary in width as they extend along absorbent core 320a.

Absorbent core 320a may also include a confluence region 332 where central channel 326, first lateral channel 328, and second lateral channel 330 come together. As shown, the optional confluence region 332 may be at least substantially circular such that a reference circle 332A encompasses a majority (e.g., substantially all) of confluence region 332. Reference circle 332A is drawn to intersect at least two (e.g., all four) of confluence points 332B, 332C, 332D, and 332E. Confluence points 332B, 332C, 332D, and 332E are points along central channel 326 where confluence region 332 starts (or ends), which correspond to points along central channel 326 where first and second lateral channels 328, 330 start to divert. For example, confluence points 332B and 332C correspond to points on central channel 326 where first lateral channel 328 starts to divert, while confluence points 332D and 332E correspond to points on central channel 326 where second lateral channel 330 starts to divert. In other configurations, a central confluence region can be considered the region circumscribed by the largest circle, centered on a longitudinal axis of the longest one of the intersecting channels (e.g., channel 326 in the embodiment of FIG. 3) that does abut at least one anterior boundary of the intersecting channels and at least one posterior boundary of the intersecting channels. Of course, in an absorbent core comprising fluff pulp, the confluence points will not be so precise after manufacturing of the core, and assembly and packaging of the article.

As illustrated, confluence region 332 may have a center and a transverse dimension. Like in the implementation shown, the center of confluence region 332 may correspond to the center of reference circle 332E and/or the transverse dimension of confluence region 332 may corresponds to the diameter of reference circle 332E. Confluence region 332 can be positioned such that its center is aligned with the insult point to improve distribution and containment of fluids in absorbent core 320a and/or absorbent article 300. The center of confluence region 332 can be, for example, at a distance from a longitudinal midpoint of absorbent core 320a that is greater than or equal to, or between two of, 5, 10, 15, 20, 25, 30, 35, 40 millimeters (e.g., between 10 and 30 millimeters), as non-limiting examples. The lateral dimension (diameter) of confluence region 332 can be, for example, greater than or equal to, or between two of, 10, 15, 20, 25, 30, 35, 40, 45, 50, 55, 60, 65, 70 millimeters (e.g., between 20 and 60 millimeters), as non-limiting examples. As will be appreciated by those of skill in the art, the boundaries may not be precise after, for example, assembly, packaging, and/or shipping.

Absorbent core 320a may include any material or combination of materials suitable for absorbing liquids, such as, for example, a laminate (e.g., a single layer laminate or a multi-layer laminate). The laminate may be formed from a nonwoven fabric or material. "Nonwoven" fabrics, according to an INDA (Association of the Nonwoven Fabrics Industry) definition, are broadly defined as sheet or web structures bonded together by entangling fiber or filaments, and by perforating films, mechanically, thermally, or chemically. Nonwoven fabrics are flat, porous sheets that are made directly from separate fibers or from molten plastic or plastic film. Nonwoven fabrics are not made by weaving or knitting and do not require converting the fibers to yarn. The basis weight of nonwoven fabrics is usually expressed as grams per square meter (gsm).

Absorbent core 320a may preferably also contain materials like super absorbent particles (SAP) and/or cellulosic fibers that are configured to absorb liquid in absorbent article 300. "Superabsorbent" or "superabsorbent material" or "SAP" refers to a water-swellable, water-insoluble organic or inorganic material capable, under the most favorable conditions, of absorbing at least about 15 times its weight in an aqueous solution containing 0.9 weight percent sodium chloride and, more desirably, at least about 30 times its weight in an aqueous solution containing 0.9 weight percent sodium chloride and, even more desirably, at least about 50 times its weight in an aqueous solution containing 0.9 weight percent sodium chloride. Illustrative superabsorbent polymer material suitable for use in absorbent core 320a can include any superabsorbent polymer particles known from superabsorbent literature, for example such as described in Modern Super-absorbent Polymer Technology, F. L. Buchholz, A. T. Graham, Wiley 1998. For example, the SAP particles may be spherical, spherical-like irregularly shaped particles, such as sausage shaped particles, or ellipsoid shaped particles of the kind typically obtained from inverse phase suspension polymerizations. The SAP particles can also be optionally agglomerated at least to some extent to form larger particles. In some implementations, the SAP particles can also have surface modifications, such as a partial or full surface coating or crosslinking, for example to increase the gel strength of the SAP particles.

The SAP materials can be natural, synthetic, and modified natural polymers and materials. In addition, the SAP materials can be or include organic compounds such as cross linked polymers. "Cross-linked" is a commonly understood term and refers to any approach for effectively rendering normally water-soluble materials substantially water insoluble, but swellable. Such polymers can include, for example, carboxymethylcellulose, alkali metal salts of polyacrylic acids, polyacrylamides, polyvinyl ethers, hydroxypropyl cellulose, polyvinyl morpholinone, polymers and copolymers of vinyl sulfonic acid, polyacrylates, polyacrylamides, polyvinyl pyridine and the like. Other suitable polymers include hydrolyzed acrylonitrile grafted starch, acrylic acid grafted starch, and isobutylene maleic anhydride copolymers, and mixtures thereof. Organic high-absorbency materials can include natural materials, such as agar, pectin, guar gum and peat moss. In addition to organic materials, superabsorbent materials may also include inorganic materials, such as absorbent clays and silica gels. Suitable examples of SAP include T9030, T9600, T9900, and Saviva polymers from BASF Corporation in Charlotte, North Carolina; and W211, W112A, W125, S125D, QX-W1482, QX-W1486, QX-W1504, and QX W1505 from Nippon Shokubai Co. Ltd, N.A.I.I. in Houston, Texas; and AQUA KEEP SA50 II, SA55SX II, SA60N II, SA65S, HP500E, HP600, and HP 700E from Sumitomo Seika Chemicals Co., Ltd. in Osaka, Japan.

For example, the SAP can have a centrifuge retention capacity of 20-60 grams per gram (g/g), for example 30-50 g/g or 33-52 g/g, optionally between 33 and 38 g/g, or optionally between 44 and 48 g/g, as non-limiting examples. The SAP can have particle size distribution (PSD) with most or substantially all particles having a diameter between 150 micrometers (µm) and 850 µm. Preferably, all or substantially all of the SAP particles in at least one of the absorbent laminate have a diameter less than or equal to 500 µm to reduce the roughness of the absorbent laminate. For example, ones of the SAP particles in absorbent core 320a having a diameter greater than or equal to 500 µm can account for less than 10% (e.g., less than 3% or less than 0.2%) of the mass of the SAP particles in the core. An illustrative SAP suitable for absorbent core 320a is HP500E from Sumitomo Seika Chemicals Co., Ltd. in Osaka, Japan. As used herein, particle diameter refers to the equivalent diameter of the particle if the particle is modelled as a sphere.

In some implementations, the SAP material of the absorbent laminate can be disposed within a matrix of adhesive material. Suitable adhesive material can include, for example, a thermoplastic hot-melt adhesive composition or a pressure-sensitive thermoplastic adhesive composition. For example, an absorbent lamina can include at least 90% (e.g., greater than 93% or 94%), by weight, SAP and less than or equal to 10% (e.g., less than 6% or 7%), by weight, adhesive. To illustrate, the SAP of the absorbent lamina can have a basis weight of at least 40 grams per square meter (gsm), such as, for example, greater than or equal to or between any two of 40, 50, 60, 70, 80, 90, 100 or more gsm (e.g., between 60 and 75 gsm).

Thus, FIG. 3 depicts an absorbent article 300 with an optional absorbent core 320a, which includes an optional central channel 326 and optional lateral channels 328, 330 that are substantially free of absorbent material. These channels help direct fluids to non-used portions of absorbent core 320a, help maintain stability of absorbent core 320a, and assist with form fitting to the wearer of absorbent article 300. For example, these channels may direct fluids toward the front and sides of absorbent core 320a, which improves the fluid distribution and leakage protection of absorbent article 300. The channels also give direct and immediate contact to any wetness indicator located near one of the channels. Positioning these channels near the crotch point of the wearer (e.g., adjacent to the wearer's crotch area) reduces or minimizes the crotch width of absorbent article 300 during use.

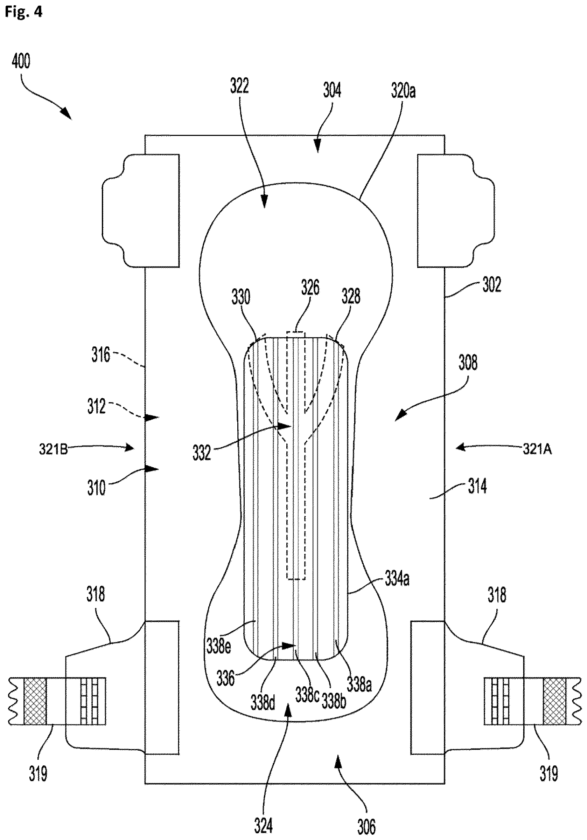

Referring to FIG. 4, an absorbent article 400 is shown. Absorbent article 400 may be a disposable absorbent article, such as an incontinence brief, protective underwear, pad, diaper, training pant, and the like, as illustrative, non-limiting examples. Absorbent article 400 is substantially similar to absorbent article 300, with the exception that article 400 includes an optional additional absorbent core (334a) disposed between absorbent core 320a and topsheet 316. Like absorbent core 320a, absorbent core 334a may include any material or combination of materials suitable for absorbing liquids; such as, for example, laminates, super absorbent particles (SAP), and/or cellulosic fibers that are configured to absorb liquid.

The optional additional absorbent core 334a can be coupled to absorbent core 320a and can, but need not, extend longitudinally along the entire length of absorbent core 320a. For example, absorbent core 334 can have a longitudinal length at least, or between any two of, 10%, 20%, 30%, 40%, 50%, 60%, 70%, or 80% smaller than a length of absorbent core 320a, and optionally be positioned closer to rear waist portion 306 than to front waist portion 304. A longitudinal length of absorbent core 334a can be, for example, greater than or equal to, or between two of, 90, 120, 150, 180,210, 240, 270, 300, 330, 360, 390, 420, 450, 480, 510, 540, 560, 590, 620, 650, 680, 710, 740, 770, 800 millimeters (e.g., between 300 and 451 millimeters), as non-limiting examples. Absorbent core 334a can cover a portion of central channel 326, a portion of lateral channel 328, and/or a portion of lateral channel 330. In the implementation illustrated in FIG. 4, absorbent core 334a covers a majority (e.g., substantially all) of central channel 326, lateral channel 328, and lateral channel 330. In such implementation shown in FIG. 4, a front portion of central channel 326, a front portion of lateral channel 328, and/or a front portion of lateral channel 330 extends from under absorbent core 334a (from a top view perspective).

As shown, absorbent core 334a may include one or more embossed elements or regions. An optional embossed pattern 336 may have one or more embossed linear paths 438 extending longitudinally from a rear half of absorbent core 334a to a front half of absorbent core 334a. Like in the implementation illustrated in FIG. 4, absorbent core 334a may include embossed pattern 336 with five (or any other number of) embossed linear paths 338a, 338b, 338c, 338d, 338d. Embossed pattern 336 may overlies a portion of central channel 326, and/or a portion of lateral channel 328, and/or a portion of lateral channel 330. More particularly, as shown, embossed pattern 336 may have an embossed linear path 338c that overlies a portion of central channel 326; and/or two embossed linear paths 338a, 338b that overlie a portion of first lateral channel 328; and/or two embossed linear paths 338d, 338e that overlie a portion of second lateral channel 330. Like in the depicted example, the centerline of embossed linear path 338c may align with the centerline of central channel 326. Other implementations may have other embossed regions and/or embossed patterns (e.g., embossed patterns including other embossed elements, such as curved and/or angled paths).

Together with absorbent core 320a, the optional additional absorbent core 334a can provide multiple benefits compared to conventional absorbent articles and/or cores. For example, embossed pattern 336 can direct fluids within absorbent core 334a to regions to the front and rear of absorbent core 334a. Embossed elements (e.g., embossed linear paths 338) can also help manage and disperse the initial fluid flow (e.g., surge) at the interface between absorbent core 334a and topsheet 316 (e.g., at the surface of absorbent core 334a). Embossed pattern 336 can direct fluids to the channels (e.g., 326, 328, and 330) of absorbent core 320a, which may, in turn, direct fluids to the front and sides of absorbent core 320a, which improves the fluid distribution and leakage protection of absorbent article 400. Also, embossed pattern 336 can further assist in establishing a preferred contoured shape of absorbent article 400 during use, which improves fit of absorbent article 400.

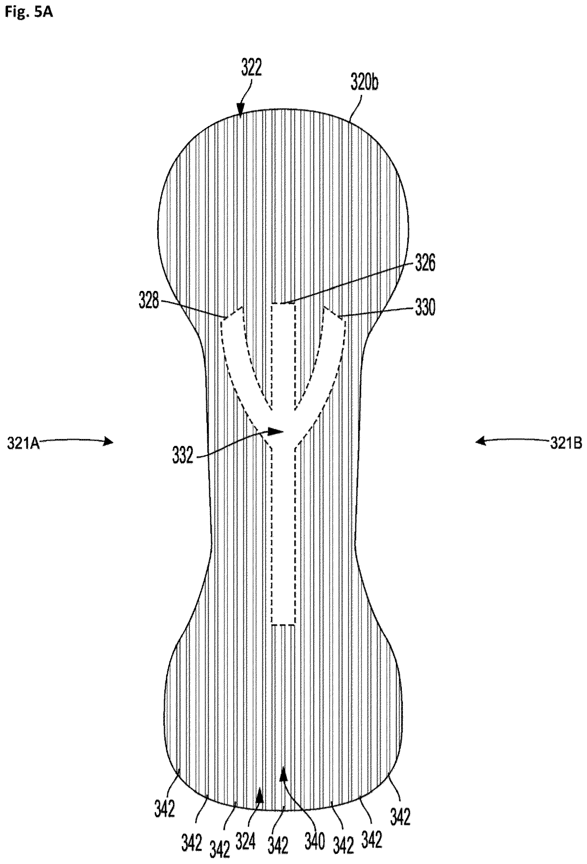

Referring to FIG. 5A, an absorbent core 320b is shown. Absorbent core 320b may be included in a disposable absorbent article, such as incontinence brief, protective underwear, pad, diaper, training pant, and the like, as illustrative, non-limiting examples.

Absorbent core 320b is substantially similar to absorbent core 320a, with the exception that absorbent core 320b additionally includes one or more optional embossed elements or regions. As shown, embossed pattern 340 may include one or more embossed linear paths 342 that may preferably extend longitudinally along absorbent core 320b. Central channel 326, lateral channel 328, and lateral channel 330 are preferably located between different portions and/or elements of embossed pattern 340. Like in the depicted example, central channel 326, lateral channel 328, and lateral channel 330 may be between a front portion of one or more embossed linear paths 342 and/or a rear portion of the one or more embossed linear paths 342. And in the depicted example, central channel 326, lateral channel 328, and lateral channel 330 are between an embossed linear path 342 near the left side of absorbent core 320b and an embossed linear path 342 near the right side of absorbent core 320b, being preferred, but optional. As shown, embossed pattern 340 may include an embossed linear path 342 with a centerline that aligns with the centerline of central channel 326. Other implementations may have other embossed regions and/or embossed patterns (e.g., embossed patterns including other embossed elements, such as curved and/or angled paths).

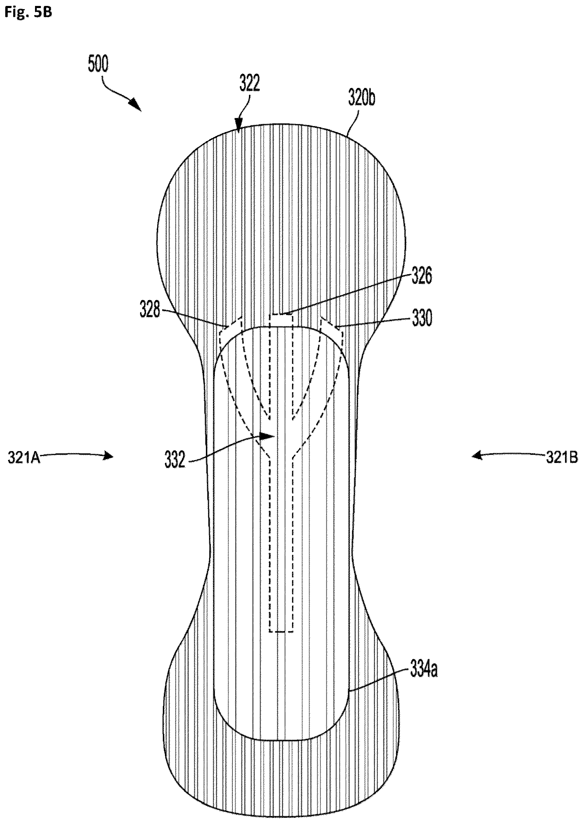

Referring to FIG. 5B, an absorbent core assembly 500 is shown. Absorbent core assembly 500 may be included in a disposable absorbent article, such as incontinence brief, protective underwear, pad, diaper, training pant, and the like, as illustrative, non-limiting examples. Like in the example of FIG. 5B, absorbent core assembly 500 may include absorbent core 320b and/or absorbent core 334a. The embossed elements and/or regions in these absorbent cores can help disperse fluids along the absorbent cores, which improves the fluid distribution and leakage protection of the absorbent cores and/or article.

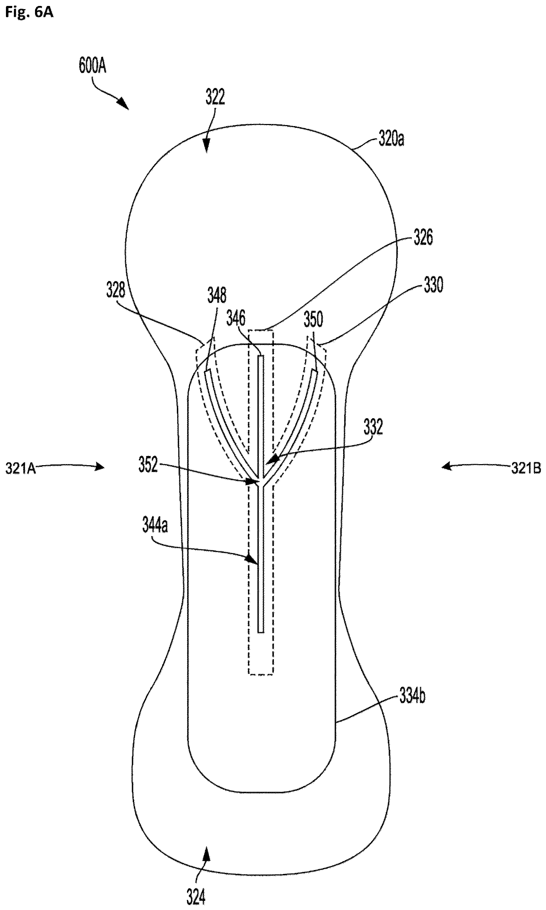

Referring to FIG. 6A, an absorbent core assembly 600A is shown. Absorbent core assembly 600A may be included in a disposable absorbent article, such as an incontinence brief, protective underwear, pad, diaper, training pant, and the like, as illustrative, non-limiting examples. Absorbent core assembly 600A includes absorbent core 320a and absorbent core 334b. Absorbent core 334b is similar to absorbent core 334a, with the exception that, instead of embossed pattern 336, absorbent core 334b includes an optional embossed pattern 344a.

Like in the depicted example, absorbent core 334b may include one or more embossed elements or regions. Embossed pattern 344a may include an embossed central path and/or one or more embossed lateral paths. Like in the example depicted in FIG. 6A, embossed pattern 344a may include embossed central path 346, and/or first embossed lateral path 348, and/or second embossed lateral path 350. As shown, first and second embossed lateral paths 348, 350 may extend along respective curved paths that extend laterally outward from a point 352 on embossed central path 346 toward a side edge of the absorbent core, and/or extend longitudinally toward an end of the absorbent core. First and second embossed lateral paths 348, 350 can be referred to as front embossed lateral paths because the majority of their respective lengths is in the front half of absorbent core 334b. As shown in the depicted example, embossed central path 346 may be centered relative to the side edges of absorbent core 334b.

Embossed pattern 344a may overlie a portion of central channel 326, and/or a portion of first lateral channel 328, and/or a portion of second lateral channel 330. For example (see FIG. 6A), a portion of embossed central path 346 may overlie a portion of central channel 326, and/or a portion of first embossed lateral path 348 may overlie a portion of first lateral channel 328 and/or a portion of central channel 326, and/or a portion of second embossed lateral path 350 may overlie a portion of second lateral channel 330 and a portion of centra channel 326. As shown, in the example of FIG. 6A, the centerline of embossed central path 346 may align with the centerline of central channel 326, and/or the centerline of first embossed lateral path 348 may aligns with the centerline of first lateral channel 328, and/or the centerline of second embossed lateral path 350 may align with the centerline of second lateral channel 330. Other implementations may have other embossed regions and/or embossed patterns (e.g., embossed patterns including other embossed elements, such as straight, curved, and/or angled paths). For example, in other configurations, the embossed pattern may have a first embossed central path that overlies a portion of a central channel, and/or a second embossed central path that overlies a portion of a first lateral channel, and/or a third embossed central path that overlies a portion of a first lateral channel. As another example, in other configurations, a portion of one or more of embossed lateral paths may overlie a portion of a central channel, a portion of a first lateral channel, and/or a portion of a second lateral channel.

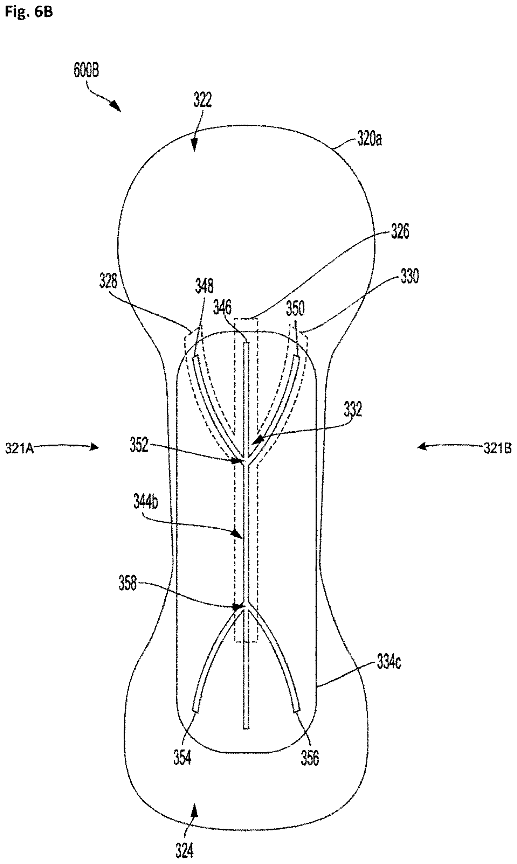

Referring to FIG. 6B, an absorbent core assembly 600B is shown. Absorbent core assembly 600B may be included in a disposable absorbent article, such as an incontinence brief, protective underwear, pad, diaper, training pant, and the like, as illustrative, non-limiting examples. Absorbent core assembly 600B may include absorbent core 320a and/or absorbent core 334c. Absorbent core 334c is similar to absorbent core 334b, with the exception that, instead of embossed pattern 344a, absorbent core 334c includes embossed pattern 344b.

As shown in the depicted example, absorbent core 334c may include one or more embossed elements and/or regions. Embossed pattern 344b may include an embossed central path and/or one or more embossed lateral paths. Embossed pattern 344b is similar to embossed pattern 344a, but includes, in addition (optionally), third embossed lateral path 354 and fourth embossed lateral path 356. As shown, third and fourth embossed lateral paths 354, 356 may extend along respective curved paths that extend laterally outward from a point 358 on embossed central path 346 toward a side edge of the absorbent core, and/or may extend longitudinally toward an end of the absorbent core. Third and fourth embossed lateral paths 354, 356 can be referred to as rear embossed lateral paths because the majority of their respective lengths is in the rear half of absorbent core 334c. Like in the depicted example, embossed central path 346 may be centered relative to the side edges of absorbent core 334c.

Embossed pattern 344b may overlie a portion of central channel 326, and/or a portion of first lateral channel 328, and/or a portion of second lateral channel 330. Like in FIG. 6B, a portion of embossed central path 346 may overlie a portion of central channel 326, and/or a portion of first embossed lateral path 348 may overlie a portion of first lateral channel 328 and/or a portion of central channel 326, and/or a portion of second embossed lateral path 350 may overlie a portion of second lateral channel 330 and/or a portion of centra channel 326, and/or a portion of third embossed lateral path 354 and/or a portion of fourth embossed lateral path 356 may overlie a portion of central channel 326. As shown, in the example of FIG. 6B, the centerline of embossed central path 346 may align with the centerline of central channel 326, and/or the centerline of first embossed lateral path 348 may align with the centerline of first lateral channel 328, and/or the centerline of second embossed lateral path 350 may align with the centerline of second lateral channel 330. Other implementations may have other embossed regions and/or embossed patterns (e.g., embossed patterns including other embossed elements, such as straight, curved, and/or angled paths).

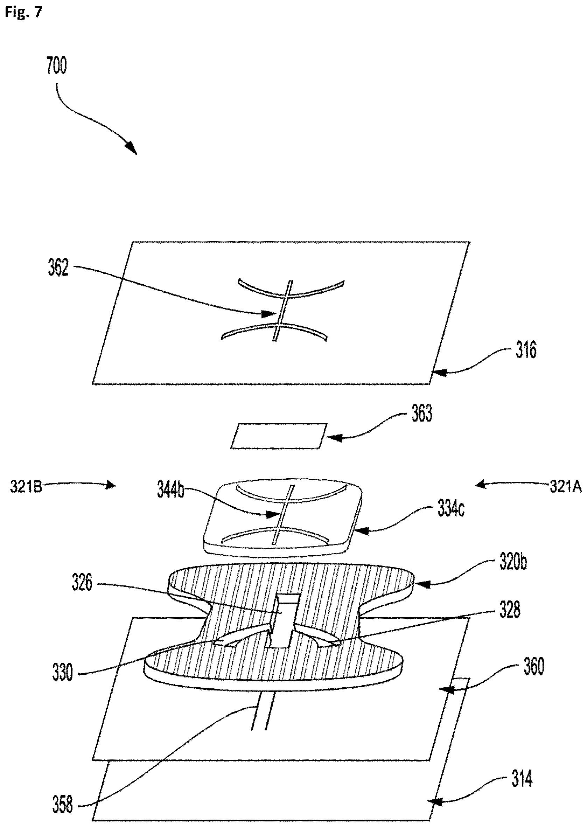

Referring to FIG. 7, an absorbent article 700 is shown. Absorbent article 700 may be a disposable absorbent article, such as an incontinence brief, protective underwear, pad, diaper, training pant, and the like, as illustrative, non-limiting examples.

Absorbent article 700 may include at least one of a liquid-impermeable backsheet 314, a liquid permeable topsheet 316, an absorbent core 320b positioned between topsheet 316 and backsheet 314, an absorbent core 334c positioned between absorbent core 320b and topsheet 316, a wetness indicator 358 positioned between absorbent core 320b and backsheet 314, a storage core (or containment layer) 360 positioned between absorbent core 320b and backsheet 314, a marking 362 on topsheet 316, and an acquisition-distribution layer ADL 363 positioned between absorbent core 320b and topsheet 316.

The wetness indicator 358 may be, like in the depicted example, disposed between absorbent core 320b and containment layer 360. Wetness indicator 358 may be coupled to backsheet 314 and/or containment layer 360. Wetness indicator 358 may be configured to indicate a wetness of absorbent core 320b. For example, wetness indicator 358 may be configured to change color when the amount of fluid contained by absorbent core 320b satisfies a threshold. As another example, wetness indicator 358 may be configured to display graphics or text on backsheet 314 when the amount of fluid contained by absorbent core 320b satisfies a threshold. Any type of wetness indicator may be used.

Topsheet 316 may include a marking 362. Like in the depicted example, the marking 362 may have a pattern substantially similar to and substantially aligned with the channels of absorbent core 320b. As shown, marking 362 may have a pattern including at least one of a central line, a first lateral curve, a second lateral curve, a third lateral curve, and a fourth lateral curve. Like in the depicted example, a portion of the central line may be aligned with (overlies) at least one of a portion of central channel 326, a portion of the first lateral curve is aligned with a portion of first lateral channel 328, and a portion of the second lateral curve is aligned with a portion of second lateral channel 330. But the marking may have any pattern. For example, in other configurations, the marking may have a pattern substantially similar to and substantially aligned with embossed pattern 344b. Marking 362 may be made and/or coupled to topsheet 316 in any way. For example, marking 362 may be painted on topsheet 316, marking 362 may be embossed on topsheet 316, and/or marking 362 may be attached to topsheet 316 using, for example, an adhesive.

ADL 363 may be (see FIG. 7), disposed between topsheet 316 and absorbent core 334c. ADL 363 may be rectangle and may have a center corresponding to the center of the rectangle. Like in the shown configuration, ADL 363 may be configured to overlie the confluence region of absorbent core 320b. The center of ADL 363 may be substantially aligned with the center of the confluence region of absorbent core 320b. While conventional absorbent articles may include ADLs exceeding 200 millimeters in length (longitudinally), absorbent articles may include an ADL with a length under 200 millimeters at least in part due to the alignment of the ADL with the confluence region of the absorbent core. For example, the longitudinal length of ADL 363 can be, for example, greater than or equal to, or between two of, 120, 130, 140, 150, 160, 170, 180, 190, 200, 210, 220, 230, 240 (e.g., between 150 and 195 millimeters), as non-limiting examples. As another example, ADL 363 can have a longitudinal length at least, or between any two of, 30%, 40%, 50%, 60%, 70%, 80%, or 90% smaller than a length of absorbent core 320b (e.g., between 50% and 80% smaller than a length of the absorbent core). ADL 363 may have any shape and may include any material or combination of materials suitable for receiving and distributing liquids, such as, for example, a laminate (e.g., a single layer laminate or a multi-layer laminate). The laminate may be formed from a nonwoven fabric or material. Additional layers for fluid management, reinforcement, and performance enhancement may be positioned between the topsheet and the backsheet.

Thus, FIG. 7 describes absorbent article 700 with wetness indicator 358, storage core 360, marking 362, and ADL 363 (all being optional, but preferred features). The optional wetness indicator 358 helps wearers of absorbent article 700 identify when absorbent article 700 has been used and/or needs to be replaced. The optional storage core 360 helps with containment of excess fluids. Optional marking 362 helps wearers of absorbent article 700 position absorbent article 700 so that the confluence region of absorbent core 320b is preferably substantially aligned with the insult point, which improves distribution and containment of fluids in absorbent article 700. And optional ADL 363 helps distribute liquids before they reach absorbent core 334c and/or absorbent core 320b, which prevents oversaturation of absorbent cores 334c, 320b and improves the absorbent characteristics of absorbent article 700 and/or cores 334c, 320b, including strikethrough and rewet characteristics. As shown in FIG. 7 all the optional features are preferably combined, but they do no need to.

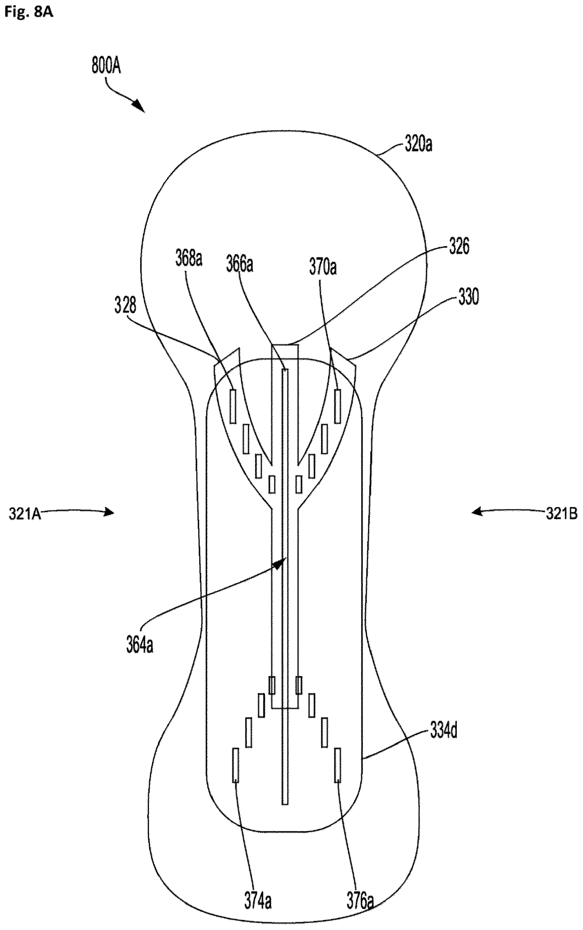

Referring to FIG 8A, absorbent core assembly 800A is shown. Absorbent core assembly 800A may be included in a disposable absorbent article, such as an incontinence brief, protective underwear, pad, diaper, training pant, and the like, as illustrative, non-limiting examples. Absorbent core assembly 800A may include absorbent core 320a and/or absorbent core 334d. Absorbent core 334d is similar to absorbent core 334a, with the exception that, instead of embossed pattern 336, absorbent core 334d may include embossed pattern 364a. Embossed pattern 364a may include an embossed central path and one or more embossed lateral paths.

Like in the example depicted in FIG. 8A, embossed pattern 364a may include one embossed central path 366a; and/or four (or any other number of) first embossed lateral paths 368a on the front left side of absorbent core 334d; and/or four (or any other number of) second embossed lateral paths 370a on the front right side of absorbent core 334d; and/or four (or any other number of) third embossed lateral paths 374a on the rear left side of absorbent core 334d; and/or four (or any other number of) fourth embossed lateral paths 376a on the rear right side of absorbent core 334d. As shown, first, second, third, and fourth embossed lateral paths 368a, 370a, 374a, 376a may be linear paths that may extend longitudinally along absorbent core 334e. Moreover, preferably each series of lateral paths may be positioned along a curved route that extends laterally outward from embossed central path 366a toward a side edge of absorbent core 334d and extends longitudinally toward an end of absorbent core 334d. For example, the series of first embossed lateral paths 368a may be positioned along a curved route substantially similar to the curved path of first lateral channel 328. Additionally, the length of each lateral path may increase as the distance from embossed central path 366a increases. For example, the first embossed lateral path 368a that is closest to embossed central path 366a may be shorter than the first embossed lateral path 368a that is second closest to embossed central path 366a (and so forth). Embossed pattern 364a overlies a portion of central channel 326, a portion of first lateral channel 328, and a portion of second lateral channel 330. Like in the depicted example, a portion of embossed central path 366a may overlie a portion of central channel 326, and/or a portion of a first embossed lateral path 368a overlies a portion of first lateral channel 328 and/or a portion of central channel 326. A portion of a second embossed lateral path 370a may overlie a portion of second lateral channel 330 and/or a portion of central channel 326, and/or a portion of a third and/or a fourth embossed lateral paths 374a, 376a may overlie a portion of central channel 326. As shown, in the example of FIG. 8A, the centerline of embossed central path 366a may align with the centerline of central channel 326.

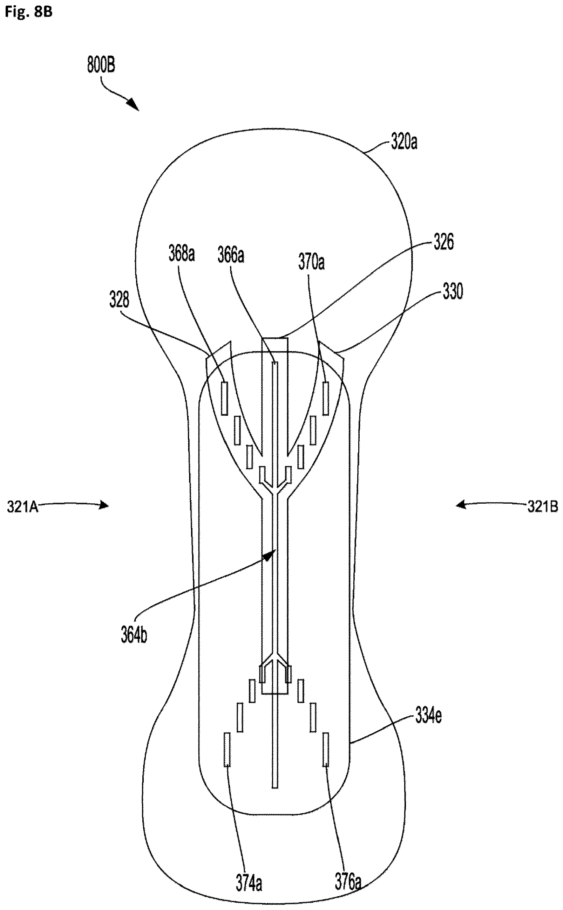

Referring to FIG. 8B, absorbent core assembly 800B is shown. Absorbent core assembly 800B may be included in a disposable absorbent article, such as an incontinence brief, protective underwear, pad, diaper, training pant, and the like, as illustrative, non-limiting examples. Absorbent core assembly 800B may include absorbent core 320a and/or absorbent core 334e. Absorbent core 334e is similar to absorbent core 334d, with the exception that, instead of embossed pattern 364a, absorbent core 334e includes embossed pattern 364b. Embossed pattern 364b includes an embossed central path and one or more embossed lateral paths. Embossed pattern 364b is similar to embossed pattern 364a, but (in embossed pattern 364b) embossed central path 366a is additionally connected to at least one of each embossed lateral path 368a, 370a, 374a, 376a.

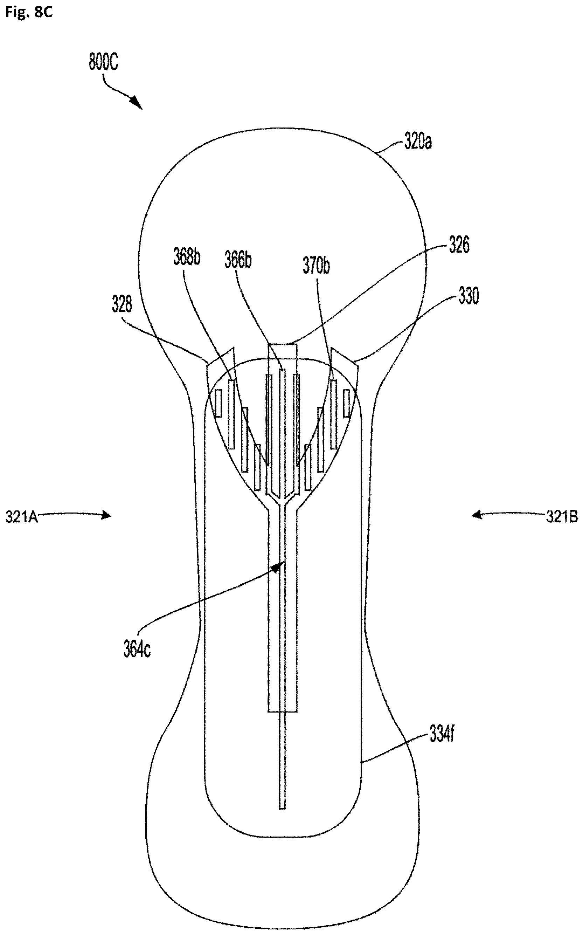

Referring to FIG. 8C, absorbent core assembly 800C is shown. Absorbent core assembly 800C may be included in a disposable absorbent article, such as an incontinence brief, protective underwear, pad, diaper, training pant, and the like, as illustrative, non-limiting examples. Absorbent core assembly 800C may include absorbent core 320a and/or absorbent core 334f. Absorbent core 334f is similar to absorbent core 334a, with the exception that, instead of embossed pattern 336, absorbent core 334f includes embossed pattern 364c. Embossed patterns 364c includes an embossed central path and one or more embossed lateral paths.

Like in the example depicted in FIG. 8C, embossed pattern 364c may include one embossed central path 366b; five (or any other number of) first embossed lateral paths 368b on the front left side of absorbent core 334f; and five (or any other number of) second embossed lateral paths 370b on the front right side of absorbent core 334f. As shown, first and second embossed lateral paths 368b, 370b may be linear paths that may extend longitudinally along absorbent core 334f. Moreover, each series of lateral paths 368b, 370b may be positioned along a curved route that extends laterally outward from embossed central path 366b toward a side edge of absorbent core 334f and/or extends longitudinally toward an end of absorbent core 334f. For example, the series of first embossed lateral paths 368b may be positioned along a curved route substantially similar to the curved path of first lateral channel 328. As shown in FIG. 8C, embossed central path 366b may be connected to at least one of each embossed lateral path 368b, 370b. Embossed pattern 364c may overlie a portion of central channel 326, and/or a portion of first lateral channel 328, and/or a portion of second lateral channel 330. As can be seen in FIG. 8C, a portion of embossed central path 366b may overlie a portion of central channel 326, and/or a portion of a first embossed lateral path 368b may overlie a portion of first lateral channel 328 and/or a portion of central channel 326, and/or a portion of a second embossed lateral path 370b may overlie a portion of second lateral channel 330 and/or a portion of central channel 326. As shown, in the example of FIG. 8C, the centerline of embossed central path 366b may align with the centerline of central channel 326.

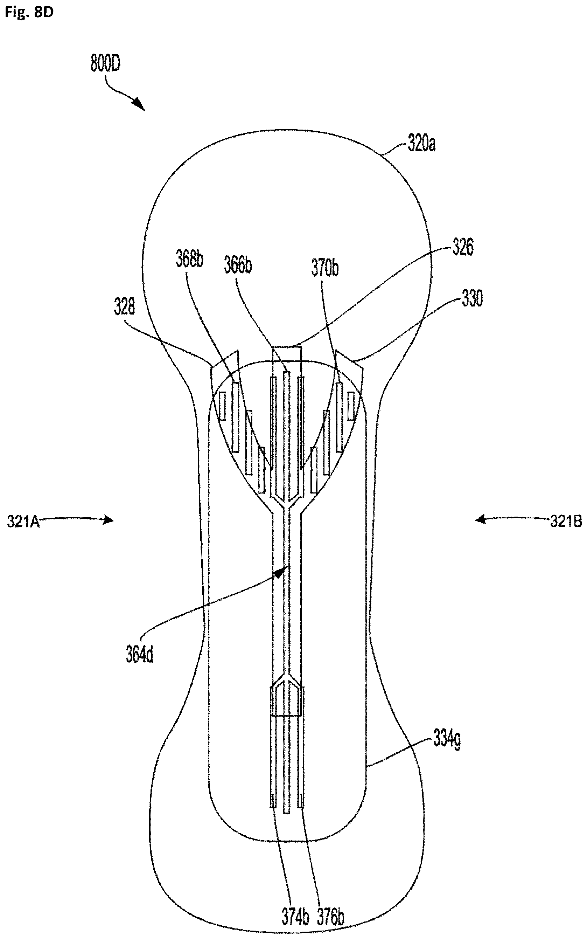

Referring to FIG. 8D, absorbent core assembly 800D is shown. Absorbent core assembly 800D may be included in a disposable absorbent article, such as an incontinence brief, protective underwear, pad, diaper, training pant, and the like, as illustrative, non-limiting examples. Absorbent core assembly 800D includes absorbent core 320a and absorbent core 334g. Absorbent core 334g is similar to absorbent core 334f, with the exception that, instead of embossed pattern 364c, absorbent core 334g is shown with and may hence include embossed pattern 364d. Embossed pattern 364d may include an embossed central path and one or more embossed lateral paths.

Embossed pattern 364d is similar to embossed pattern 364c, but (embossed pattern 364d) additionally includes one third embossed lateral path 374b on the rear left side of absorbent core 334g and one fourth embossed lateral path 376b on the rear right side of absorbent core 334g. As shown, first, second, third, and fourth embossed lateral paths 368b, 370b, 374b, 376b may be linear paths that extend longitudinally along absorbent core 334g. As can be seen in FIG. 8D, embossed central path 366b may be connected to at least one of each lateral path 368b, 370b, 374b, 376b. Embossed pattern 364d may overlie a portion of central channel 326, and/or a portion of first lateral channel 328, and/or a portion of second lateral channel 330. Like in FIG. 8D, a portion of embossed central path 366b may overlie a portion of central channel 326, and/or a portion of a first embossed lateral path 368b may overlie a portion of first lateral channel 328 and/or a portion of central channel 326, and/or a portion of a second embossed lateral path 370b may overlie a portion of second lateral channel 330 and/or a portion of central channel 326, and a portion of third and/or fourth embossed lateral paths 374b, 376b may overlie a portion of central channel 326. As shown, in the example of FIG. 8D, the centerline of embossed central path 366b may align with the centerline of central channel 326.

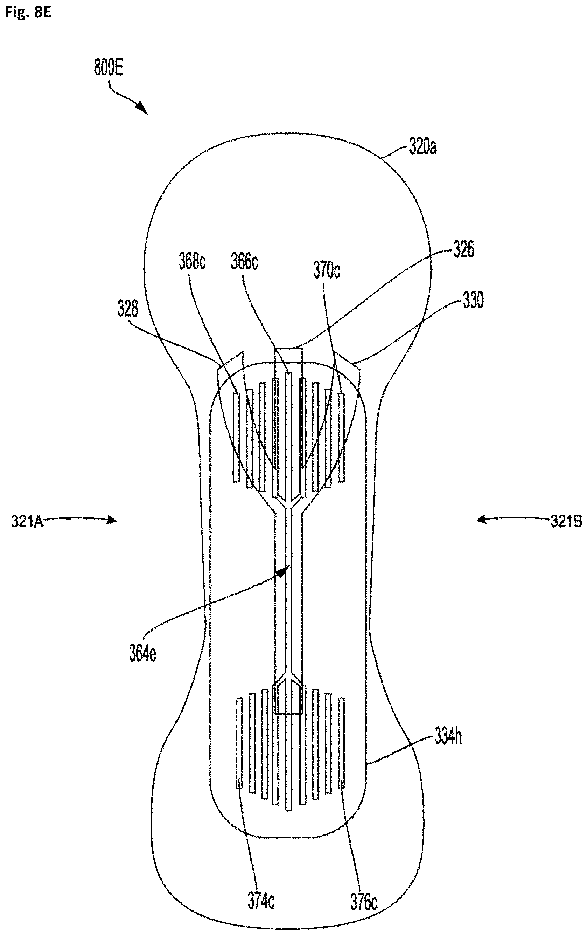

Referring to FIG. 8E, absorbent core assembly 800E is shown. Absorbent core assembly 800E may be included in a disposable absorbent article, such as an incontinence brief, protective underwear, pad, diaper, training pant, and the like, as illustrative, non-limiting examples. Absorbent core assembly 800E may include absorbent core 320a and absorbent core 334h. Absorbent core 334h is similar to absorbent core 334a, with the exception that, instead of embossed pattern 336, absorbent core 334h is shown with and may hence include embossed pattern 364e. Embossed pattern 364e may include an embossed central path and one or more embossed lateral paths.

As shown in FIG. 8E, embossed pattern 364e may include at least one of one embossed central path 366c; four (or any other number of) first embossed lateral paths 368c on the front left side of absorbent core 334h; four (or any other number of) second embossed lateral paths 370c on the front right side of absorbent core 334h; four (or any other number of) third embossed lateral paths 374c on the rear left side of absorbent core 334h; and four (or any other number of) fourth embossed lateral paths 376c on the rear right side of absorbent core 334h. As shown, first, second, third, and fourth embossed lateral paths 368c, 370c, 374c, 376c may be linear paths that extend longitudinally along absorbent core 334h. Moreover, the length of each lateral path may decrease as the distance from embossed central path 366c increases. For example, the first embossed lateral path 368c that is closest to embossed central path 366c may be longer than the first embossed lateral path 368c that is second closest to embossed central path 366c (and so forth). Like in the example depicted in Fig. 8E, embossed central path 366c may be connected to at least one of each lateral path 368c, 370c, 374c, 376c. Embossed pattern 364e may overlie a portion of central channel 326, and/or a portion of first lateral channel 328, and/or a portion of second lateral channel 330. As shown, a portion of embossed central path 366c may overlie a portion of central channel 326, and/or a portion of a first embossed lateral path 368c may overlie a portion of first lateral channel 328 and/or a portion of central channel 326, a portion of a second embossed lateral path 370c may overlie a portion of second lateral channel 330 and/or a portion of central channel 326, and/or a portion of a third and a fourth embossed lateral paths 374c, 376c may overlie a portion of central channel 326. As shown, in the example of FIG. 8E, the centerline of embossed central path 366c may aligns with the centerline of central channel 326.

Other implementations may have other embossed regions and/or embossed patterns (e.g., embossed patterns including other embossed elements, such as straight, curved, and/or angled paths).

The above specification and examples provide a complete description of the structure and use of illustrative implementations. Although certain examples have been described above with a certain degree of particularity, or with reference to one or more individual examples, those skilled in the art could make numerous alterations to the disclosed implementations without departing from the scope of this invention. As such, the various illustrative implementations of the methods and systems are not intended to be limited to the particular forms disclosed. Rather, they include all modifications and alternatives falling within the scope of the claims, and examples other than the one shown may include some or all of the features of the depicted example. For example, elements may be omitted or combined as a unitary structure, and/or connections may be substituted. Further, where appropriate, aspects of any of the examples described above may be combined with aspects of any of the other examples described to form further examples having comparable or different properties and/or functions, and addressing the same or different problems. Similarly, it will be understood that the benefits and advantages described above may relate to one embodiment or may relate to several implementations.

The claims are not intended to include, and should not be interpreted to include, means plus- or step-plus-function limitations, unless such a limitation is explicitly recited in a given claim using the phrase(s) "means for" or "step for," respectively.

## Claims

1. An absorbent article comprising:
- a chassis having a front waist portion, a rear waist portion, and a crotch portion between the front and rear waist portions, the chassis having a length extending from a front waist edge of the front waist portion to a rear waist edge of the rear waist portion, the chassis comprising:
∘ a liquid-permeable topsheet, and
∘ a liquid-impermeable backsheet; and
- an absorbent first core disposed between the topsheet and the backsheet, the first core comprising a mixture of cellulosic fibers, super-absorbent polymer (SAP), and adhesive, the first core having a front end, a rear end, two side edges, a length extending longitudinally between the front end and the rear end, a front half between a longitudinal midpoint of the first core and the front end, and a rear half between the longitudinal midpoint of the first core and the rear end,
**characterized in, that** the first core defines:
- a central channel extending longitudinally from the rear half to the front half, and
- two lateral channels in the front half of the first core, the lateral channels extending from a common point on the central channel toward respective ones of the side edges of the first core;
and **in that** the first core is not enclosed in a core cover within the topsheet and backsheet.

2. The absorbent article of claim 1, **characterized in that**:
(i) the two lateral channels are front lateral channels extending longitudinally toward the front end of the first core, and/or
(ii) the common point is within a confluence region, and the confluence region has a first transverse dimension of from 20 to 60 mm.

3. The absorbent article of claim 2(ii), **characterized in that** the confluence region is:
(i) circular and the first transverse dimension is a diameter, and/or
(ii) located in the front half and has a center that is 10 to 30 mm from the longitudinal midpoint of the first core.

4. The absorbent article of any of claims 1-3, **characterized in that** it further comprises an absorbent second core disposed between the first core and the topsheet and covering at least a portion of the central channel and a portion of the two lateral channels.

5. The absorbent article of claim 4, **characterized in that** the second core comprises an embossed first pattern, having:
(i) one or more embossed linear paths extending longitudinally from a rear half of the second core to a front half of the second core, and/or
(ii) an embossed central path extending longitudinally from a rear half of the second core to a front half of the second core, and two embossed lateral paths extending from a common point on the embossed central path toward respective ones of side edges of the second core.

6. The absorbent article of claim 5, **characterized in that** at least a portion of at least one of the embossed linear paths and/or a portion of the embossed central path overlies at least a portion of the central channel and in case of alternative (ii) at least a portion of each of the embossed lateral paths overlies at least a portion of a corresponding one of the lateral channels.

7. The absorbent article of any of claims 5(ii) or 6(ii), **characterized in that** the two embossed lateral paths are embossed front lateral paths extending from the common point toward a front end of the second core.

8. The absorbent article of claim any of claims 5(ii) or 7, **characterized in that** the embossed first pattern further comprises two embossed rear lateral paths extending from a rear common point on the embossed central path toward a rear end of the second core.

9. The absorbent article of any of claims 1-8, **characterized in that**
(i) the absorbent article further comprises a storage core disposed between the first core and the backsheet, and/or
(ii) the first core further comprises one or more embossed linear paths extending longitudinally from a rear half of the first core to a front half of the first core, and/or
(iii) the topsheet comprises a marking having a second pattern, and at least a portion of the second pattern is substantially aligned with at least a portion of the central channel and at least a portion of the two lateral channels, and/or
(iv) at least one of the central channel and the two lateral channels has a transverse dimension of from 10 to 25 mm.

10. The absorbent article of claim 9(iv), **characterized in that** the transverse dimension is of 25 mm.

11. The absorbent article of any of claims 5-10, **characterized in that** at least one of an embossed linear path, an embossed central path, and an embossed lateral path has a transverse dimension of from 3 to 8 mm.

12. The absorbent article of any of claims 1-11, further comprising an acquisition-distribution layer.

13. The absorbent article of claim 12, **characterized in that** the acquisition-distribution layer and/or a center of the acquisition-distribution layer overlies the common point on the central channel and/or **in that** the acquisition-distribution layer is disposed between the topsheet and the first core.

14. The absorbent article of any of claims 12 or 13, **characterized in that** a longitudinal dimension of the acquisition-distribution layer is of (i) less than 200 mm and/or (ii) from 120 to 170 mm and/or (iii) more than 75 or 78 percent smaller than a longitudinal dimension of the first core.
